# EUROPÄISCHE PATENTSCHRIFT

(11) **EP 1 882 484 B1**
(45) Veröffentlichungstag und Bekanntmachung des Hinweises auf die Patenterteilung: **05.08.2009**
(21) Anmeldenummer: 07113129.6
(22) Anmeldetag: 25.07.2007
(51) Int. Cl.: A61M 1/10, A61M 1/12

(54) **Übertragungsanordnung**
Transmission assembly
Agencement de transmission

(30) Priorität: 27.07.2006 DE 102006035547
(43) Veröffentlichungstag der Anmeldung: 30.01.2008
(73) Patentinhaber: Deutsches Zentrum für Luft- und Raumfahrt e.V., 51147 Köln (DE)
(72) Erfinder: VODERMAYER, Bernhard, 82205, Gilching (DE); SCHMID, Thomas, 82205, Gilching (DE)
(74) Vertreter: von Kirschbaum, Alexander

(56) Entgegenhaltungen:
- US-A- 4 550 731
- US-A- 5 314 453
- US-A1- 2005 131 486
- YOSHINORI MITAMURA ET AL: "A TRANSCUTANEOUS OPTICAL INFORMATION TRANSMISSION SYSTEM FOR IMPLANTABLE MOTOR-DRIVEN ARTIFICIAL HEARTS" ASAIO TRANSACTIONS, HARPER AND ROW PUBLISHERS, HAGERSTOWN, MD, US, Bd. 36, Nr. 3, 1. Juli 1990 (1990-07-01), Seiten 278-280, XP000204533
- EIJI OKAMOTO ET AL: "Development of a bidirectional transcutaneous optical data transmission system for artificial hearts allowing long-distance data communication with low electric power consumption" JOURNAL OF ARTIFICIAL ORGANS ; THE OFFICIAL JOURNAL OF THE JAPANESE SOCIETY FOR ARTIFICIAL ORGANS, SPRINGER-VERLAG, TO, Bd. 8, Nr. 3, 1. September 2005 (2005-09-01), Seiten 149-153, XP019374429 ISSN: 1619-0904

## Beschreibung

Die Erfindung bezieht sich auf eine Übertragungs-Anordnung mit einer intrakorporalen Übertragungsspule eines Implantates und einer extrakorporalen Übertragungsspule eines extrakorporalen Versorgungsgerätes.

Medizinische Implantate, beispielsweise zur Unterstützung von Herz- und Organfunktionen, können grundsätzlich teilimplantiert oder vollimplantiert werden. Teilimplantierte Implantate weisen zur Energieversorgung und für die Datenübermittlung Leitungen auf, die durch die Haut zu einem extrakorporalen Versorgungsgerät geführt sind, Die Leitungs-Durchführungen in der Haut stellen einen bevorzugten Infektionsherd dar. Ferner wird der Patient durch die Leitungs-Durchführungen in seiner Bewegungsfreiheit eingeschränkt.

Eine Alternative für teilimplantierte Implantate sind vollimplantierte Implantate, Insbesondere Implantate, die mechanische Arbeit verrichten, beispielsweise Blutpumpen, haben einen Energiebedarf von 2 bis 20 Watt. Dieser kann allenfalls kurzfristig für maximal wenige Stunden aus einem implantateigenen Akkumulator zur Verfügung gestellt werden. Zum Betrieb von Implantaten mit einem Energieverbrauch von mehr als 1 bis 2 Watt ist eine dauerhafte oder nahezu dauerhafte extrakorporale Energieversorgung unerlässlich, Die Energieversorgung eines derartigen Implantates sowie die Datenübertragung erfolgt leitungsfrei durch eine intrakorporale Versorgungsspule des Implantates und eine extrakorporale Übertragungsspule des Versorgungsgerätes. Die Energie- und die Datenübertragung erfolgen induktiv zwischen den beiden Übertragungsspulen. Zur Daten- und Energieübertagung zwischen dem Versorgungsgerät und dem Implantat werden die beiden Übertragungsspulen möglichst gut einander überdeckend aufeinander gelegt. Das extrakorporale Versorgungsgerät ist häufig als Gürtel oder als Tasche ausgelegt und bezieht die auf das Implantat zu übertagende Energie aus Batterien oder aus dem öffentlichen Stromnetz.

Um einen möglichst hohen Wirkungsgrad bei der Energie- und Datenübertragung zu erzielen, müssen die beiden Übertragungsspulen so exakt wie möglich übereinander platziert und anschließend entsprechend zueinander fixiert werden. Die Fixierung erfolgt beispielsweise durch Festkleben des Versorgungsgerätes bzw. seiner Übertragungsspule auf der Haut.

Es sind Übertragungs-Anordnungen bekannt, die einen guten Patienten-Komfort bieten und auf eine klebende Fixierung des Versorgungsgerätes bzw. seiner Übertragungsspule verzichten. Beispielsweise kann die Versorgungsgerät-Übertragungsspule in einem Gürtel untergebracht sein, der vom Patienten leicht entfernt werden kann. Wegen der fehlenden Klebfixierung auf der Haut besteht jedoch während der induktiven Übertragung die Gefahr, dass bei Patienten-Bewegungen der Gürtel und die Versorgungsgerät-Übertragungsspule verrutschen und sich damit die elektromagnetische Kopplung und damit der Wirkungsgrad der Energieübertagung und der Datenübertragung verschlechtern.

Ein weiteres Problem stellt die genaue Platzierung und Ausrichtung der extrakorporalen Versorgungsgerät-Ubertragungsspule über bzw. auf der intrakorporalen Implantat-Übertragungsspule dar. In der Praxis muss hierzu die Position der intrakorporalen Implantat-Übertragungsspule ertastet werden. Wenn sich die Implantat-Übertragungsspule nicht ertasten lässt, ist auf der Patienten-Haut an der betreffenden Stelle eine entsprechende Markierung vorzusehen.

Aus US 4,550,731 ist es bekannt, einen intrakorporalen Herzschrittmacher mit einer extrakorporalen Steuer- oder Auswerteeinheit induktiv zu koppeln. Zur richtigen Positionierung der Steuer- oder Auswerteeinheit sind LED's vorgesehen, die die Position der extrakorporalen Einheit relativ zum Herzschrittmacher anzeigen, wobei die LED's stufenweise von einem Blinkzustand in ein Dauerleuchten übergehen, wenn die extrakorporale Einheit näher an den Herzschrittmacher bewegt wird und schließlich richtig über dem Herzschrittmacher positioniert ist.

Aufgabe der Erfindung ist es, eine Übertragungs-Anordnung zu schaffen, die eine Kontrolle der Ausrichtung der Implantat-Übertagungsspule mit der Versorgungsgerät-Übertagungsspule erlaubt.

Diese Aufgabe wird erfindungsgemäß gelöst mit den Merkmalen des Patentanspruches 1.

Die erfindungsgemäße Übertragungs-Anordnung weist einen der Implantat-Übertragungsspule fest zugeordneten Ortungs-Sender und einen der Versorgungsgerät-Übertragungsspule fest zugeordneten Ortungs-Empfänger auf. Ferner ist ein dem Versorgungsgerät zugeordnetes Auswertungsmodul vorgesehen, das mit dem Empfänger verbunden ist und in Abhängigkeit von dem Versatz und/oder der Versatzrichtung von Sender und Empfänger zueinander ein Lokalisierungssignal ausgibt.

Erfindungsgemäß weist der Empfänger mehrere Empfangselemente auf, wobei das Lokalisierungssignal eine Versatzrichtung angibt, in der die Übertragungsspulen zueinander versetzt sind.

Der Sender und der Empfänger arbeiten jeweils mit der selben Wellenart, beispielsweise mit Lichtwellen, mit elektromagnetischen Feldern, mit Funkwellen oder mit akustischen Wellen . Das mit dem Empfänger verbundene Auswertungsmodul kann aus der Intensität des von dem Sender kommenden Signals den Versatz, also die Entfernung und/oder die Versatzrichtung also den Raum- oder Ebenen-Winkel des Versatzes des Senders zum Empfänger feststellen und in Form des Lokalisierungssignals ausgeben.

Das Lokalisierungssignal kann akustisch, optisch oder auf andere Weise ausgegeben werden und versetzt den Patienten in die Lage, die Position der Versorgungsgerät-Übertragungsspule so zu korrigieren, dass die Versorgungsgerät-Übertagungsspule mit der Implantat-Übertragungsspule wieder weitgehend zur Deckung kommt. Durch die Kontrollierbarkeit des Versatzes der Implantat-Übertragungsspule zu der Versorgungsgerätkann der Versatz ständig kontrolliert und gering gehalten werden. Hierdurch verbessert sich der Wirkungsgrad bzw. die Effizienz der Übertragung, insbesondere der Energieübertragung zwischen dem Versorgungsgerät und dem Implantat. Hierdurch wiederum wird die Wärmeentwicklung verringert, die Haltbarkeit der beteiligten Komponenten verbessert, und, im Falle von Datenübertragung, die Qualität der Datenübertagung verbessert.

Grundsätzlich ist auch eine umgekehrte Anordnung von Sender und Empfänger denkbar, also ein extrakorporaler Sender, der dem Versorgungsgerät zugeordnet ist und ein intrakorporaler Empfänger, der dem Implantat zugeordnet ist. Die von dem Empfänger empfangenen Signale müssen dann zur Auswertung bzw. zur Ausgabe des Lokalisierungssignals von dem Implantat zu dem Versorgungsgerät übertragen werden.

Das Lokalisierungssignal kann ggf. auch dazu verwendet werden, die Versorgungsgerät-Übertragungsspule automatisch, d. h. mit Hilfe entsprechender Stellmotoren wieder optimal mit der Implantat-Übertagungsspule auszurichten.

Die Übertragungsspulen dienen der Energieübertragung können jedoch ergänzend oder alternativ auch der Datenübertragung zwischen dem Versorgungsgerät und dem Implantat dienen.

Vorzugsweise ist ein Senderelement des Senders eine Spule und der Empfänger weist ein Magnetfeld-Empfangselement auf. Das Empfangselement kann seinerseits eine Spule sein, oder als Hallsensor ausgebildet sein, Das Senderelement kann die Implantat-Übertragungsspule sein, kann jedoch auch eine separate Senderelement-Spule sein. Das oder die Senderelemente sind mit dem oder den Empfängerelementen durch ein elektromagnetisches Feld verbunden. Die durch das Empfängerelement bzw. die Empfängerelemente festgestellte Stärke des Feldes ist ein Maß für die Entfernung des als Spule ausgebildeten Empfängerelements. Die Empfangsspule ist bevorzugt im Umfang erheblich kleiner als die Versorgungsgerät-Übertragungsspule bzw. die Implantat-Übertragungsspule. Hierdurch kann die Ausrichtung der beiden Übertragungsspulen miteinander erheblich genauer festgestellt werden.

Alternativ zu einer induktiven Ausbildung des Empfängerelementes und des Senderelementes können diese auch optisch ausgebildet sein. Das Senderelement ist dann eine Lichtquelle, die Licht im sichtbaren und/oder unsichtbaren Bereich ausstrahlt. Das Empfängerelement ist ein optisches Empfangselement, das das von dem Senderelement ausgestrahlte Licht empfängt und mengenmäßig erfasst. Besonders geeignet ist Infrarot-Licht, da die Haut und das Unterhautgewebe im Bezug auf Infrarot-Licht eine relativ niedrige Absorption aufweist. Als Empfangselement kommt insbesondere ein Fototransistor oder eine Fotodiode in Frage.

Das optische Sendeelement kann beispielsweise genau mittig in der intrakorporalen Übertragungsspule des Implantates angeordnet sein. Für eine Bestimmung der Versatzrichtung über den Versatz hinaus sind bevorzugt mindestens drei Empfangselemente vorgesehen, die beispielsweise in Form eines gleichseitigen Dreieckes zueinander angeordnet sind. Neben dem ungefähren Versatz kann dann auch die ungefähre Versatzrichtung ermittelt werden.

Gemäß einer weiteren bevorzugten Ausgestaltung weist der Sender mehrere Senderelemente auf, deren Sendesignale verschieden voneinander sind. Beispielsweise können als Senderelemente Leuchtdioden unterschiedlicher Farben bzw. Wellenlängen eingesetzt werden. Ferner muss das Empfangselement in der Lage sein, die von den Sendeelementen ausgestrahlte Strahlung zu differenzieren, d. h. im Falle von optischen Sendeelementen wellenlängenselektiv zu empfangen. Wird hierzu ein einziges ein Spektrum generierendes Empfangselement verwendet, so kann auf diese Weise mit einem einzigen Empfangselement die Versatzrichtung festgestellt werden.

Werden mindestens zwei wellenlängenselektive Empfangselemente verwendet, kann ferner die rotartorische Position der Implantat-Ubertragungsspule zu der Versorgungsgerät-Übertragungsspule festgestellt werden.

Die von den Empfangselementen empfangenen Signale werden in einem Mikrocomputer bzw. Mikrocontroller ausgewertet und es wird ein entsprechenden Lokalisierungssignal von dem Mikrocomputer bzw. Mikrocontroller ausgegeben.

Da Daten zwischen dem Implantat und dem Versorgungsgerät häufig ohnehin über Infrarot-Senderelemente und Infrarot-Empfängerelemente ausgetauscht werden, kann in diesem Fall die optische Variante mit nur geringen oder ggf. ohne jeden Zusatzaufwand für die Hardware realisiert werden.

Um Störungen durch externe Lichtquellen auszuschließen, können die optischen Signale moduliert werden, beispielsweise gepulst werden. Über seine Pulsfrequenz lässt sich das optische Sendelement auf der Empfängerseite eindeutig identifizieren und von dem Licht anderer Lichtquellen unterscheiden. Ist das Sendeelement der vorliegenden Vorrichtung gleichzeitig ein Sendelement für den optischen Datenaustausch zwischen dem Implantat und dem Versorgungsgerät, arbeitet die Versatzerkennung nicht kontinuierlich, sondern beispielsweise getaktet.

Im folgenden werden unter Bezugnahme auf die Zeichnungen mehrere Ausführungsbeispiele der Erfindung näher erläutert.

Es zeigen:
- Fig. 1: eine Übertragungs-Anordnung mit elektromagnetischen Sender- und Empfängerelementen in einer Prinzipskizze,
- Fig. 2: die Übertragungs-Anordnung der Fig. 1 im Querschnitt,
- Fig. 3: ein zweites Ausführungsbeispiel einer Übertagungs-Anordnung mit optischen Sende- und Empfangselementen, und
- Fig. 4: die Anordnung von optischen Sende- und Empfangselementen eines dritten Ausführungsbeispiels einer Übertragungs-Anordnung.

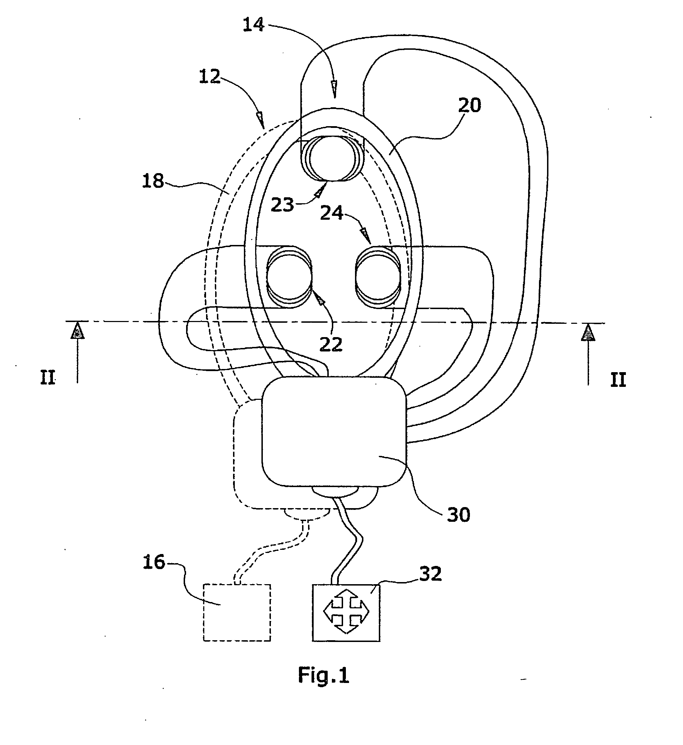

In der Fig. 1 ist ein erstes Ausführungsbeispiel einer Übertragungs-Anordnung 10 dargestellt. Die Übertragungs-Anordnung 10 besteht aus einem in unterbrochenen Linien dargestellten intrakorporalen Implantat 12 und einem extrakorporalen Versorgungsgerät 14. Das Implantat 12 ist beispielsweise eine Blutpumpe zur Unterstützung des Patienten-Herzens und weist eine Pumpeinrichtung 16 auf. Die Pumpeinrichtung 16 läut in der Regel kontinuierlich und hat einen Energieverbrauch von mehreren Watt bis zu 20 Watt. Das Implantat 12 ist ein sogenanntes Vollimplantat, d. h. das Implantat 12 weist keinerlei physische Verbindung zur Körperaußenseite auf.

Die elektrische Energie für den Betrieb der Blutpumpe 16 muss daher schnurlos übertragen werden. Dies erfolgt durch eine Übertragungsspule 18 des Implantates 12, die knapp unterhalb der Haut implantiert ist, und eine extrakorporale Übertragungsspule 20 des extrakorporalen Versorgungsgerätes 14. Innerhalb der Übertragungsspule 20 des Versorgungsgerätes 14 sind als Empfangselemente 22, 23, 24 kleine Spulen angeordnet, die induktive Empfangselemente für ein wechselndes Magnetfeld bilden,

Die Implantat-Übertragungsspule 18 bildet ein Senderelement, das den Sender in Bezug auf den Empfänger bzw. die Empfangselemente 22 bis 24 des Versorgungsgerätes 14 bildet.

Besteht eine optimale Kopplung zwischen den beiden Übertragungsspulen 18, 20, dann wird während eines in der Implantat-Übertragungsspule 18 kurzzeitig generierten Wechselfeldes in den Empfangselementen 22 bis 24 jeweils ungefähr die gleiche induzierte Spannung bzw. der gleiche induzierte Strom gemessen.

Die Messung des induzierten Stroms bzw. der induzierten Spannung der Empfangselemente 22, 23, 24 erfolgt in einem Auswertungsmodul 30, das mit den drei Empfangselementen 22, 23, 24 elektrisch verbunden ist. Das Auswer-tungsmodul 30 gibt in Abhängigkeit von dem gemessenen Versatz und der gemessenen Versatzrichtung zwischen dem Senderelement 18 und den Empfangselementen 22, 23, 24 ein entsprechendes Lokalisierungssignal an eine optische Anzeige 32 aus. Die optische Lokalisierungs-Anzeige 32 ist dem Versorgungsgerät 14 zugeordnet und kann einfach von dem Patienten abgelesen werden. Hierdurch wird der Patient in die Lage versetzt, die extrakorporale Versorgungsgerät-Übertragungsspule 20 bestmöglich mit der intrakorporalen Implantat-Übertragungsspule 18 auszurichten.

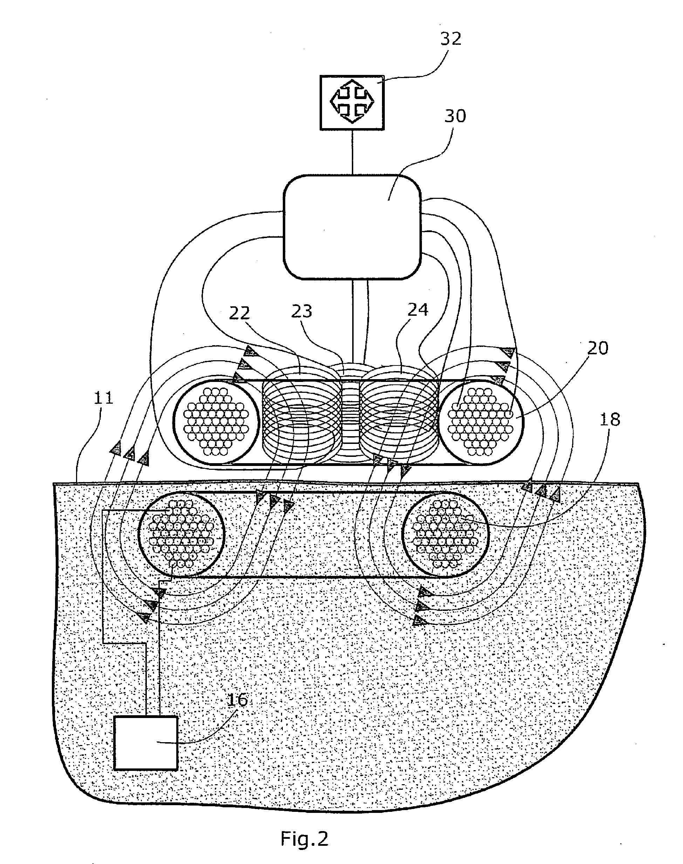

Wie in Fig. 2 dargestellt, ist die Übertragungsspule 18 des Implantates 12 subkutan, d. h. knapp unterhalb der Außenhaut 11 angeordnet.

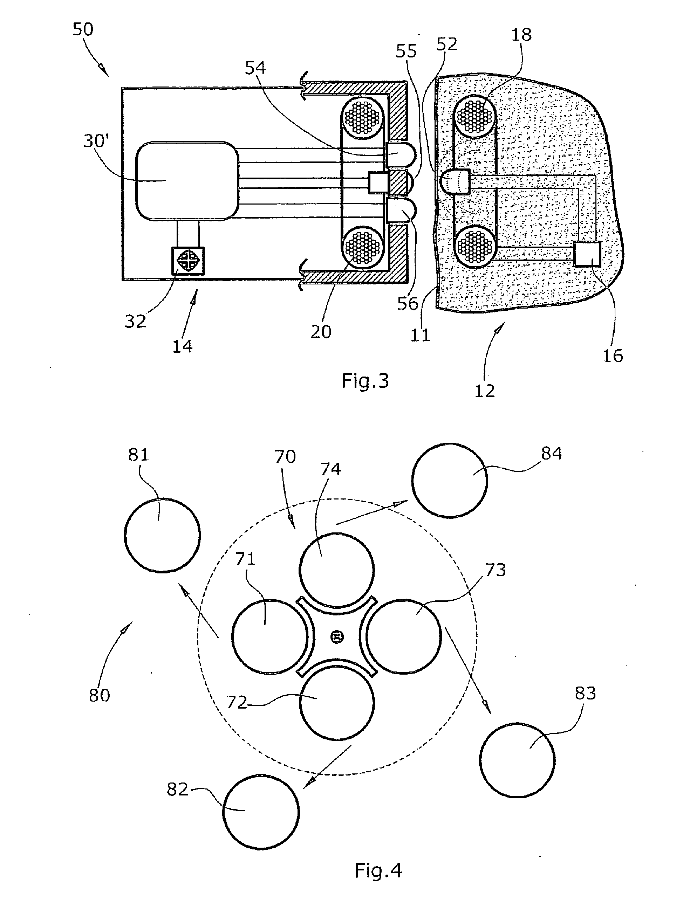

In der Fig. 3 ist ein zweites Ausführungsbeispie! einer Übertragungs-Anordnung 50 dargestellt, bei der der Sender von einem optischen Sendelement 52 und der Empfänger von drei optischen Empfangselementen 54, 55, 56 gebildet wird.

Das Sendelement 52 ist eine Infrarot-Diode, die gepulst betrieben wird. Die Empfangselemente 54, 55, 56 sind Foto-Dioden, die Infrarot-Filter aufweisen, In dem Auswertungsmodul 30' werden die von den optischen Empfangselementen 54 ,55, 56 empfangenen Signale mit einem Bandfilter gefiltert, der als Filterfrequenz die Puls-Frequenz aufweist, mit der das Sendeelement 52 zerhackt wird.

Durch eine Auswertung der von den Empfangselementen 54, 55, 56 empfangenen Intensität des von dem Sendelement 52 ausgestrahlten Infrarot-Lichtes kann das Auswertungsmodul 30 ein Signal an die Lokalisierungs-Anzeige 32 senden, das sowohl über die Versatzrichtung als auch über den Versatz der Größe nach zwischen der Versorgungsgerät-Übertragungsspule 18 und der Implantat-Übertragungsspule 20 Auskunft gibt.

In der Fig. 4 ist eine alternative Ausgestaltung des Senders 70 und des von den Empfangselementen 81 bis 84 gebildeten Empfängers 80 dargestellt. Die Sendeelemente 71 bis 74 sind jeweils Leuchtdioden verschiedener Farben bzw. verschiedener Wellenlänge. Das gleiche gilt für die Empfangselemente 81 bis 84, die jeweils im Bezug auf die Strahlung eines korrespondierenden Sendelementes 71 bis 74 selektiv empfindlich sind. Auf diese Weise kann mit einer entsprechenden Auswertung auch die rotartorische Position der Versorgungsgerät-Übertragungsspule mit der Implantat-Übertragungsspule festgestellt, als Lokalisierungssignal ausgegeben und bei Bedarf korrigiert werden.

## Patentansprüche

1. Übertragungs-Anordnung (10) mit
einer intrakorporalen Übertragungsspule (18) eines Implantates (12) und einer extrakorporalen Übertragungsspule (20) eines extrakorporalen Versorgungsgerätes (14),
einem der Implantat-Übertragungsspule (18) fest zugeordneten Sender,
einem der Versorgungsgerät-Übertragungsspule (20) fest zugeordneten Empfänger, und
einem dem Versorgungsgerät (14) zugeordneten Auswertungsmodul (30), das mit dem Empfänger verbunden ist und in Abhängigkeit von dem Versatz und/oder der Versatzrichtung von Sender und Empfänger zueinander ein Lokalisierungssignal ausgibt,
**dadurch gekennzeichnet, dass**
der Empfänger mehrere Empfangselemente (22 bis 24 ; 54 bis 56) aufweist und das Lokalisierungssignal eine Versatzrichtung angibt, in der die Übertragungsspulen (18, 20) zueinander versetzt sind.

2. Übertragungs-Anordnung nach Anspruch 1, **dadurch gekennzeichnet, dass** der Sender als Senderelement eine Spule (18) und der Empfänger als Empfangselement ein Magnetfeld-Empfangselement (22, 23, 24) aufweist.

3. Übertragungs-Anordnung nach Anspruch 1 oder 2, **dadurch gekennzeichnet, dass** ein Senderelement des Senders die Implantat-Übertagungsspule (18) ist.

4. Übertragungs-Anordnung nach einem der Ansprüche 1 bis 3, **dadurch gekennzeichnet, dass** das Empfangselement ein Hallsensor ist.

5. Übertragungs-Anordnung nach einem der Ansprüche 1 bis 3, **dadurch gekennzeichnet, dass** das Empfangselement (22 - 24) eine Spule ist.

6. Übertragungs-Anordnung nach Anspruch 1, **dadurch gekennzeichnet, dass** der Sender ein optisches Sendeelement (52) und der Empfänger ein optisches Empfangselement (54, 55, 56) aufweist.

7. Übertragungs-Anordnung nach einem der Ansprüche 1 bis 6, **dadurch gekennzeichnet, dass** der Sender mehrere Senderelemente (71, 72, 73, 74) aufweist, deren Sendesignale verschieden voneinander sind.

8. Übertragungs-Anordnung nach einem der Ansprüche 6 bis 7, **dadurch gekennzeichnet, dass** das optische Sendeelement (52) ein Infrarot-Sendeelement ist.

## Claims

1. A transfer assembly (10) comprising
an intracorporeal transfer coil (18) of an implant (12) and an extracorporeal transfer coil (20) of an extracorporeal supply means (14),
a transmitter permanently associated with said transfer coil (18) of said implant,
a receiver permanently associated with said transfer coil (20) of said supply means, and
an evaluation module (30) associated with said supply means (14) and connected with said receiver, said evaluation module (30) issuing a locating signal, depending on the offset and/or the direction of offset between said transmitter and said receiver,
**characterized in that**
said receiver comprises a plurality of receiving elements (22 to 24; 54 to 56), and said locating signal indicates a direction of offset in which the transfer coils (18, 20) are offset with respect to each other.

2. The transfer assembly according to claim 1, wherein the transmitter configured as a transmitting element comprises a coil (18), and the receiver configured as a receiving element comprises a magnetic field receiving element (22,23,24).

3. The transfer assembly according to claim 1 or 2, wherein a transmitting element of the transmitter defines the transfer coil (18) of the implant (12).

4. The transfer assembly according to one of claims 1 to 3, wherein the receiving element is configured as a Hall sensor.

5. The transfer assembly according to one of claims 1 to 3, wherein the receiving element (22-24) is configured as a coil.

6. The transfer assembly according to claim 1, wherein the transmitter comprises an optical transmitting element (52), and the receiver comprises optical receiving elements (54,55,56).

7. The transfer assembly according to one of claims 1 to 6, wherein the transmitter comprises a plurality of transmitting elements (71,72,73,74) whose transmitted signals differ from each other.

8. The transfer assembly according to one of claims 6 to 7, wherein the optical transmitting element (52) is an infrared transmitting element.

## Revendications

1. Ensemble de transmission (10) comprenant
une bobine de transmission (18) intracorporelle d'un implant (12) et une bobine de transmission (20) extracorporelle d'un appareil d'alimentation (14) extracorporel,
un émetteur associé en permanence à la bobine de transmission (18) dudit implant,
un récepteur associé en permanence à la bobine de transmission (20) dudit appareil d'alimentation, et
un module d'évaluation (30) associé audit appareil d'alimentation (14), connecté avec ledit récepteur et livrant un signal de localisation en fonction du déport et/ou de la direction de déport entre l'émetteur et le récepteur,
**caractérisé en ce que**
le récepteur comprend plusieurs éléments récepteurs (22 à 24; 54 à 56), et que le signal de localisation indique une direction de déport dans laquelle sont déportées les bobines de transmission (18, 20) entre elles.

2. Ensemble de transmission selon la revendication 1, **caractérisé en ce que** l'émetteur comprend une bobine (18) comme élément émetteur et le récepteur comprend un élément récepteur de type champ magnétique (22, 23, 24) comme élément récepteur.

3. Ensemble de transmission selon la revendication 1 ou 2, **caractérisé en ce qu'**un élément émetteur dudit émetteur est la bobine de transmission (18) de l'implant.

4. Ensemble de transmission selon l'une quelconque des revendications 1 à 3, **caractérisé en ce que** l'élément récepteur est un capteur Hall.

5. Ensemble de transmission selon l'une quelconque des revendications 1 à 3, **caractérisé en ce que** l'élément récepteur (22-24) est une bobine.

6. Ensemble de transmission selon la revendication 1, **caractérisé en ce que** l'émetteur comprend un élément émetteur optique (52) et le récepteur comprend un élément récepteur optique (54, 55, 56).

7. Ensemble de transmission selon l'une quelconque des revendications 1 à 6, **caractérisé en ce que** l'émetteur comprend plusieurs éléments émetteurs (71, 72, 73, 74), dont les signaux d'émission sont différents entre eux.

8. Ensemble de transmission selon l'une quelconque des revendications 6 à 7, **caractérisé en ce que** l'élément émetteur optique (52) est un élément émetteur infrarouge.
